# EUROPEAN PATENT APPLICATION

(11) **EP 4 154 981 A1**
(43) Date of publication of application: **29.03.2023**
(21) Application number: 22189370.4
(22) Date of filing: 09.08.2022
(51) Int. Cl.: B01L 7/00

(54) **MULTICHANNEL ISOTHERMAL AMPLIFICATION SYSTEM AND OPERATION METHOD**

(30) Priority: 24.09.2021 KR 20210126319
(71) Applicant: Nanobiolife Inc., Seoul 08504 (KR)
(72) Inventor: KIM, Soo Kyung, 10110 Gimpo-si, Gyeonggi-do (KR); KOO, Cha Ryong, 08051 Seoul (KR); NAM, Dong Hoon, 16687 Suwon-si, Gyeonggi-do (KR); LEE, Dong Chul, 16330 Suwon-si, Gyeonggi-do (KR); JANG, Seong Uk, 02755 Seoul (KR); JEONG, Do Hyun, 12096 Namyangju-si, Gyeonggi-do (KR)
(74) Representative: Isarpatent

(57) **Abstract**

Disclosed are a multi-channel isothermal amplification system and an operation method thereof, the operation method of the multi-channel isothermal amplification system comprising: a first step in which sample tubes are disposed in holes formed in a line in a heating block, respectively, wherein the heating block comprises: a first heating block area in which some of the plurality of holes are formed in a line; and a second heating block area in which the rest of the plurality of holes are formed in a line, and which is disposed to be in a line with the first heating block area, wherein the first heating block area and the second heating block area are spaced apart from each other at an interval as much as one hole area between two holes; and a second step in which an optical system moves in a longitudinal direction of the first heating block area and the second heating block area, and the optical system also causes rays of light having their respective fixed wavelengths to be successively incident upon the sample tubes into the holes of the first heating block area and the holes of the second heating block, thereby detecting respective fluorescence signals of samples disposed in the sample tubes.

## Description

### BACKGROUND OF THE INVENTION

### Field of the Invention

The present invention relates to a multi-channel isothermal amplification system, and an operation method thereof, and more particularly to a multi-channel isothermal amplification system, and an operation method thereof which can obtain result values more rapidly and efficiently through the detection of fluorescence signals using rays of light having various wavelengths.

### Description of the Related Arts

According to the advent of a technology for amplification of nucleic acids, studies for diagnosing diseases and so on by detecting a bacterium, a virus, and so on have actively been carried out. The technology for the amplification of nucleic acids is a technology which is mainly used in the field of molecular biology and biotechnology, and is a method which can detect and analyze a small quantity of nucleic acid.

In general, in order to amplify nucleic acids, a polymerase chain reaction (PCR) technique of analyzing deoxyribonucleic acid (DNA) and ribonucleic acid (RNA) using a heat stabilization enzyme has widely been used.

Existing PCR repeats a process of separating single-stranded DNA from double-stranded DNA under a condition of high temperature, and combining a primer with a single strand by lowering the temperature, thereby extending a tag polymerase to the double-stranded DNA.

However, the existing PCR showing only qualitative results of DNA fragments amplified using gel-type electrophoresis from an endpoint has various problems about accuracy of quantitative detection and so on.

Thus, a real-time PCR technique capable of realizing quantitative analysis of nucleic acids by detecting intensity of fluorescence signals, which are in proportion to each concentration of the amplified nucleic acids, in real time was developed.

The real-time PCR technique has often been utilized in analyzing nucleic acids because no gel-type electrophoresis with respect to products resulting from amplification of the nucleic acids is performed, the products can be confirmed in real time during a reaction cycle, and quantitative analysis can be performed.

Demands for technologies capable of rendering more efficient and accurate diagnosis with respect to a larger number of samples by improving this conventional art are increasing.

### [Prior Art Document]

### [Patent Document]

Patent Document 1: Korean Patent No. 10-0580639 (May 9, 2006)

### SUMMARY OF THE INVENTION

The present invention has been devised for solving the aforesaid problems, and the present invention may provide a multi-channel isothermal amplification system and an operation method thereof which are capable of detecting fluorescence signals using rays of light having various wavelengths through an optical system having multi-channels with regard to a plurality of sample tubes disposed in a heating block, thereby obtaining accurate result values for several samples more rapidly and efficiently.

Furthermore, the present invention may provide the multi-channel isothermal amplification system in which a luminous source module is disposed at a lower end of the heating block in which the sample tubes are located, and a detection module configured to detect respective fluorescent signals of samples is disposed on a side surface of the heating block so that no mutual interference of the rays of light between the optical module and the detection module occurs, thereby providing the multi-channel isothermal amplification system capable of performing more accurate measurement.

Furthermore, the multi-channel isothermal amplification system according to the present invention may be configured to be more compact because the luminous source module and the detection module are integrated, thereby more improving its usability and portability.

In order to solve the aforesaid problems, an operation method of a multi-channel isothermal amplification system according to an exemplary embodiment of the present invention may comprise: a first step in which sample tubes are disposed in holes formed in a line in a heating block, respectively, wherein the heating block comprises a first heating block area in which some of the plurality of holes are formed in a line, and a second heating block area in which the rest of the plurality of holes are formed in a line, and which is disposed to be in a line with the first heating block area, wherein the first heating block area and the second heating block area are spaced apart from each other at an interval as much as one hole area between two holes; and a second step in which an optical system moves in a longitudinal direction of the first heating block area and the second heating block area which are disposed in a line, and the optical system also causes rays of light having their respective fixed wavelengths to be successively incident upon the sample tubes inserted into the holes of the first heating block area and the holes of the second heating block area, thereby detecting respective fluorescence signals of samples disposed in the sample tubes.

According to another exemplary embodiment of the present invention, the optical system may comprise four channels consisting of: a luminous source module configured to cause the rays of light to be incident from a lower part of the heating block onto sides of the sample tubes; and a detection module configured to detect the respective fluorescence signals from the samples at a side part of the heating block, and the second step may be performed in such a manner that the optical system consisting of the first channel to the fourth channel moves in the longitudinal direction of the first heating block area and the second heating block area so that the first channel to the fourth channel cause the rays of light having their respective fixed wavelengths to be successively incident upon the sample tubes inserted into the holes of the first heating block area and the holes of the second heating block area, thereby detecting the respective fluorescence signals of the samples disposed in the sample tubes.

According to the other exemplary embodiment of the present invention, the second step may be performed in such a manner that the plurality of channels of the optical system move simultaneously, wherein the first channel among the plurality of channels moves in the longitudinal directions of the first heating block area and the second heating block area which are disposed in a line, and causes the rays of light having their respective first wavelengths to be successively incident upon the sample tubes inserted into the holes of the first heating block area and the holes of the second heating block area, thereby detecting respective fluorescence signals of the samples disposed in the sample tubes; the second channel among the plurality of channels moves in the longitudinal directions of the first heating block area and the second heating block area which are disposed in a line, and causes the rays of light having their respective second wavelengths to be successively incident upon the sample tubes inserted into the holes of the first heating block area and the holes of the second heating block area, thereby detecting respective fluorescence signals of the samples disposed in the sample tubes; the third channel among the plurality of channels moves in the longitudinal directions of the first heating block area and the second heating block area which are disposed in a line, and causes the rays of light having their respective third wavelengths to be successively incident upon the sample tubes inserted into the holes of the first heating block area and the holes of the second heating block area, thereby detecting respective fluorescence signals of the samples disposed in the sample tubes; and the fourth channel among the plurality of channels moves in the longitudinal directions of the first heating block area and the second heating block area which are disposed in a line, and causes the rays of light having their respective fourth wavelengths to be successively incident upon the sample tubes inserted into the holes of the first heating block area and the holes of the second heating block area, thereby detecting respective fluorescence signals of the samples disposed in the sample tubes.

A multi-channel isothermal amplification system according to one exemplary embodiment of the present invention may comprise: a heating block in which holes are formed in a line, into the holes are inserted a plurality of sample tubes, respectively; and an optical system configured to cause rays of light having their respective different wavelengths to be incident upon the sample tubes inserted into the holes of the heating block, thereby detecting respective fluorescence signals of samples disposed in the sample tubes, wherein the heating block comprises: a first heating block area in which some of the plurality of holes are formed in a line; and a second heating area in which the rest of the plurality of holes are formed in a line, and which is spaced apart from the first heating block area at an interval as much as one hole area between two holes in the longitudinal direction of the first heating block area so as to be disposed to be in a line with the first heating block area, wherein the optical system moves in a longitudinal direction of the first heating block area and the second heating block area which are disposed in a line, and the optical system also causes rays of light having their respective fixed wavelengths to be successively incident upon the sample tubes inserted into the holes of the first heating block area and the hole of the second heating block area, thereby detecting respective fluorescence signals of samples disposed in the sample tubes.

According to another exemplary embodiment of the present invention, the multi-channel isothermal amplification system may further comprise: a radiant heat plate configured to emit heat from the heating block to the outside; and a Peltier module configured to exchange heat between the heating block and the radiant heat plate.

According to the other exemplary embodiment of the present invention, the optical system may comprise four channels consisting of: a luminous module configured to cause the rays of light to be incident from a lower part of the heating block onto sides of the sample tubes; and a detection module configured to detect the respective fluorescence signals from the samples at a side part of the heating block.

According to the other exemplary embodiment of the present invention, the radiant heat plate may comprise: a first radiant heat plate disposed at another side part opposite to the side part at which the detection module is disposed, and configured to emit heat from the first block heating area to the outside; and a second radiant heat plate disposed at the other side part opposite to the side part at which the detection module is disposed, and configured to emit heat from the second heating block area to the outside.

According to the other exemplary embodiment of the present invention, the luminous source module may comprise: a light-emitting diode (LED) configured to irradiate the rays of light; an excitation filter configured to filter out the rays of light irradiated from the LED; and a first lens configured to concentrate the rays of light filtered out by the excitation filter on the samples disposed in the sample tubes.

According to the other exemplary embodiment of the present invention, the detection module may comprise: an emission filter configured to receive the respective fluorescence signals of the samples; a second lens which the fluorescence signals filtered out by the emission filter penetrate; and a light perception sensor configured to receive the fluorescence signals coming in through the second lens.

The multi-channel isothermal amplification system, and the operation method thereof according to the present invention can obtain accurate result values for several samples more rapidly and efficiently by performing detection of the fluorescence signals using the rays of light having various wavelengths through the optical system having the multi-channels with regard to the plurality of sample tubes disposed in the heating block.

Furthermore, in the multi-channel isothermal amplification system according to the present invention, because the luminous source module is disposed at the lower end of the heating block in which the sample tubes are located, and the detection module configured to detect the respective fluorescence signals of the samples is disposed on the side surface of the heating block, the rays of light between the luminous source module and the detection module aren't mutually interfered, so the multi-channel isothermal amplification system capable of performing more accurate measurement can be provided.

Furthermore, the multi-channel isothermal amplification system according to the present invention can be configured to be more compact because the luminous source module and the detection module are integrated, thereby more improving its usability and portability.

### BRIEF DESCRIPTION OF THE DRAWINGS

FIG. 1 is a view illustrating a multi-channel isothermal amplification system according to one exemplary embodiment of the present invention.
FIG. 2 is an inner perspective view of the multi-channel isothermal amplification system according to one exemplary embodiment of the present invention.
FIG. 3 is a view illustrating a heating block of the multi-channel isothermal amplification system according to one exemplary embodiment of the present invention.
FIG. 4 is a view for more particularly describing the constitutions of the multi-channel isothermal amplification system according to one exemplary embodiment of the present invention.
FIG. 5 is a view for describing an operation method of the multi-channel isothermal amplification system according to one exemplary embodiment of the present invention.
FIG. 6 is a block diagram of the multi-channel isothermal amplification system according to one exemplary embodiment of the present invention.
FIG. 7 is a flow chart for describing an operation method of the multi-channel isothermal amplification system according to another exemplary embodiment of the present invention.
FIG. 8 is a view for describing the operation method of the multi-channel isothermal amplification system according to another exemplary embodiment of the present invention.
FIG. 9 and FIG. 10 are graphs for describing the operation method of the multi-channel isothermal amplification system according to another exemplary embodiment of the present invention.

### DETAILED DESCRIPTION OF THE INVENTION

Hereinafter, preferable exemplary embodiments of the present invention will be described in detail with reference to the accompanying drawings. In the following description with regard to embodied forms, it is to be noted that, when the detailed description of conventional functions or elements related with the present invention may make the gist of the present invention unnecessarily unclear, the detailed description thereof will be omitted. Also, it should be understood that the sizes of the constituent elements shown in the drawings may be exaggeratedly drawn, and do not mean actually applied sizes.

Hereinafter, the constitutions of a multi-channel isothermal amplification system according to one exemplary embodiment of the present invention are described with reference to the drawings.

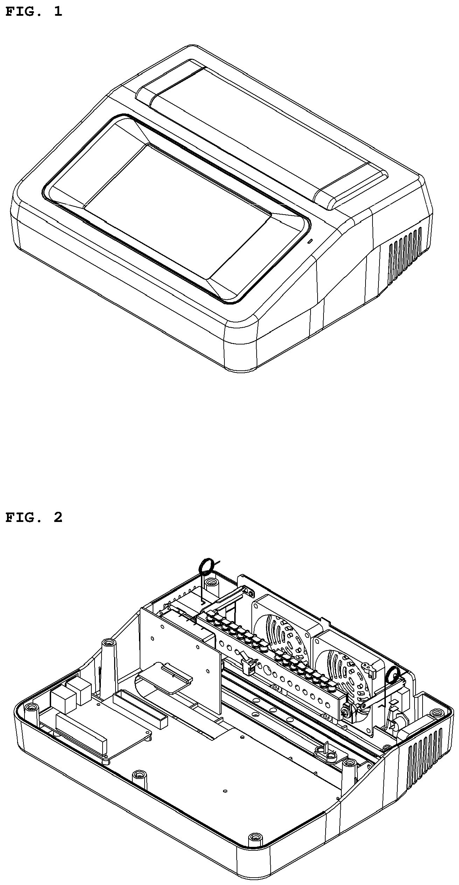

FIG. 1 is a view illustrating a multi-channel isothermal amplification system according to one exemplary embodiment of the present invention, and FIG. 2 is an inner perspective view of the multi-channel isothermal amplification system according to one exemplary embodiment of the present invention.

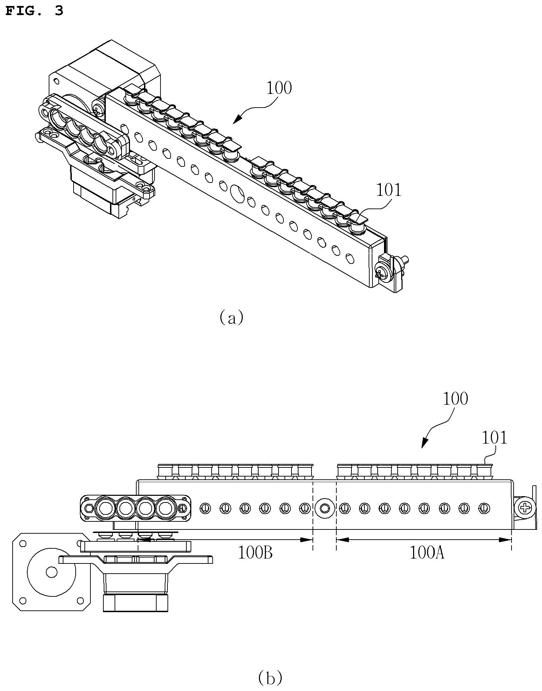

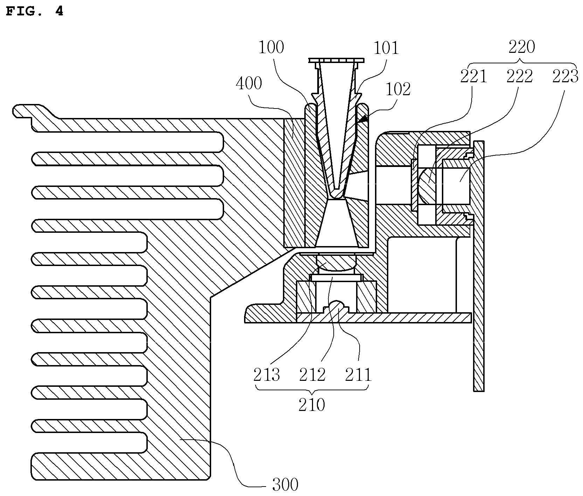

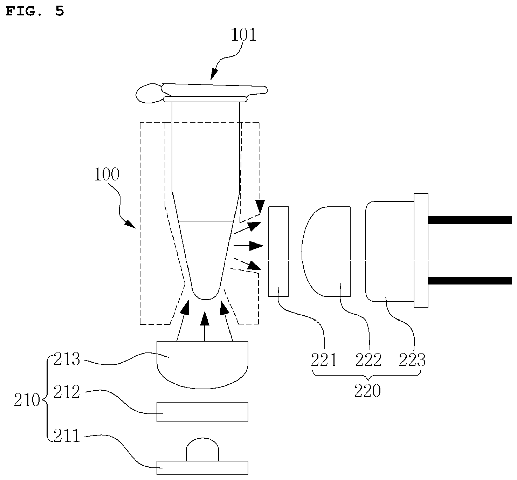

Furthermore, FIG. 3 is a view illustrating a heating block of the multi-channel isothermal amplification system according to one exemplary embodiment of the present invention, FIG. 4 is a view for more particularly describing the constitutions of the multi-channel isothermal amplification system according to one exemplary embodiment of the present invention, and FIG. 5 is a view for describing an operation method of the multi-channel isothermal amplification system according to one exemplary embodiment of the present invention.

The multi-channel isothermal amplification system according to one exemplary embodiment of the present invention comprises: a heating block 100; an optical system 200; a radiant heat plate 300; and a Peltier module 400.

Holes 102 into which a plurality of sample tubes 101 are inserted, respectively are formed in the heating block 100, and the holes 102 may be designated as wells. At this time, the holes 102 are formed in a line. That is, the holes 102 are formed in a line and in one direction in the heating block 100 so that the sample tubes 101 in which samples are received can be disposed in the holes 102, respectively.

More specifically, the heating block 100 may be divided into a first heating block area 100A and a second heating block area 100B.

Some of the plurality of holes 102 may be formed in a line in the first heating block area 100A, and in the same manner, the rest of the plurality of holes 102 may be formed in a line in the second heating block area 100B, and at this time the second heating block area 100B may be spaced apart from the first heating block area 100A at an interval as much as one hole area between two holes 102, and may be disposed to be in a line with the first heating block area 100A.

The optical system 200 causes rays of light having their respective wavelengths to be incident upon the sample tubes 101 inserted into the holes of the heating block 100, thereby detecting respective fluorescence signals of samples disposed in the sample tubes 101.

According to one exemplary embodiment of the present invention, the optical system 200 comprises four channels, wherein each of the channels may cause the rays of light having their respective different wavelengths to be incident upon the sample tubes 101 inserted into the holes of the heating block 100, respectively, may detect respective fluorescence signals of the samples disposed in the sample tubes 101, and may be configured to move in order.

More specifically, the channels of the optical system 200 may consists of a luminous source module 210 and a detection module 220.

The luminous source module 210 may cause the rays of light to be incident from a lower part of the heating block 100 onto sides of the sample tubes 101, and the detection module 220 may detect fluorescence signals from the samples at a side part of the heating block 100.

More specifically, the luminous source module 210 may comprise: a light-emitting diode (LED) 211 configured to irradiate the rays of light; an excitation filter 212 configured to filter out the rays of light irradiated from the LED 211; and a first lens 213 configured to concentrate the rays of light filtered out by the excitation filter 212 on the samples disposed in the sample tubes 101.

Furthermore, the detection module 220 may comprise: an emission filters 221 configured to receive the respective fluorescence signals of the samples; a second lens 222 which the fluorescence signals filtered out by the emission filters 221 penetrate; and a light perception sensor 223 configured to receive the fluorescence signals coming in through the second lens 222.

Meanwhile, the radiant heat plate 300 emits heat from the heating block 100 to the outside.

At this time, the radiant heat plate 300 is disposed at another side part opposite to the side part at which the detection module 220 is disposed, thereby effectively emitting the heat from the heating block 100 to the outside.

More specifically, the radiant heat plate 300 may consist of a first radiant plate disposed to correspond to the first heating block area 110A of the heating block 100, and a second radiant plate disposed to correspond to the second heating block area 110B of the heating block 100.

Furthermore, the Peltier module 400 may be disposed at a position between the heating block 100 and the radiant heat plate 300 so as to exchange heat between the heating block 100 and the radiant heat plate 300.

Like this, the multi-channel isothermal amplification system according to one exemplary embodiment of the present invention may cause the heat from the heating block 100 to be effectively cooled through the Peltier module 400, and may also cause the heat to be more effectively emitted through the radiant heat plate 300.

That is, in the multi-channel isothermal amplification system according to one exemplary embodiment of the present invention, through such a constitution, the optical system 200 consisting of the four channels may move in a longitudinal direction of the first heating block area 100A and the second heating block area 100B of the heating block 100, and each channel of the optical system 200 may cause the rays of light having their respective fixed wavelengths to be successively incident upon the sample tubes 101 inserted into the holes of the first heating block area 100A and the holes of the second heating block area 100B, thereby detecting the respective fluorescence signals of the samples disposed in the sample tubes.

Like this, the multi-channel isothermal amplification system according to one exemplary embodiment of the present invention may obtain accurate result values more rapidly and efficiently by detecting the fluorescence signals through the four channels with regard to the plurality of sample tubes disposed in the heating block.

Furthermore, according to one exemplary embodiment of the present invention, although the optical system consisting of the four channels is described as an example, the number of the channels of the optical system may be changed as needed so that rapid and efficient detection can be performed.

In addition to, in the multi-channel isothermal amplification system according to one exemplary embodiment of the present invention, the luminous source module is disposed at a lower end of the heating block in which the sample tubes are located, and the detection module configured to detect the respective fluorescence signals of the samples is disposed on a side surface of the heating block so that no rays of light between the luminous source module and the detection module are mutually interfered, and thus the multi-channel isothermal amplification system capable of performing more accurate measurement can be provided.

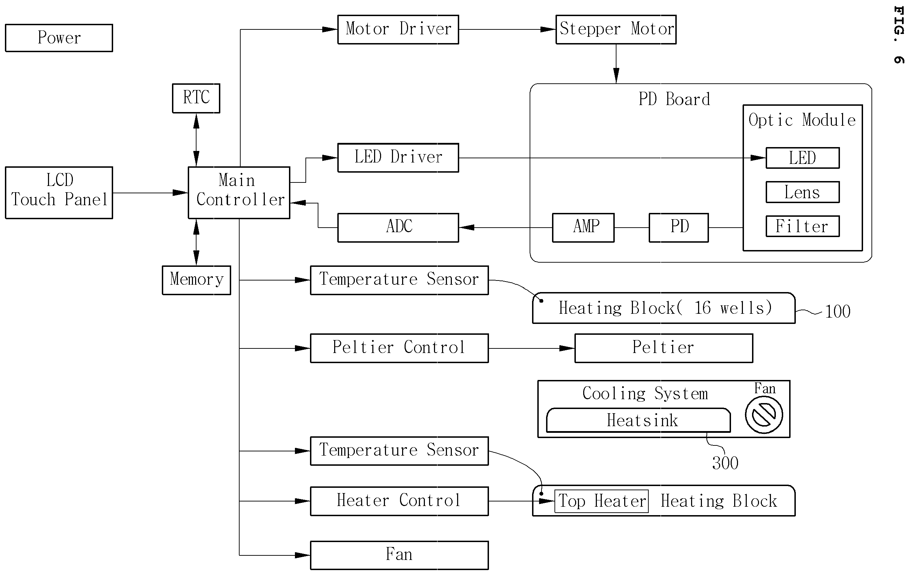

FIG. 6 is a block diagram of the multi-channel isothermal amplification system according to one exemplary embodiment of the present invention.

Referring to FIG. 6, the multi-channel isothermal amplification system according to one exemplary embodiment of the present invention may be configured to further comprise a liquid crystal digital (LCD) touch panel so that a user's control order can be inputted through the LCD touch panel, and detected information of sample signals and related information can also be displayed.

Furthermore, the multi-channel isothermal amplification system according to one exemplary embodiment of the present invention may further comprise: a main controller; a motor driver configured to cause a stepper motor to operate; a light-emitting diode (LED) driver configured to cause the LED of an optical module to operate; a temperature sensor configured to sense heat of the heating block 100; a Peltier controller configured to control the Peltier module; a heater controller configured to control a top heater installed in the heating block; a temperature sensor installed in the top heater; and a fan, thereby being controlled by the main controller.

Like this, the multi-channel isothermal amplification system according to one exemplary embodiment of the present invention may be configured to be more compact because the luminous source module and the detection module are integrated, thereby more improving its usability and portability.

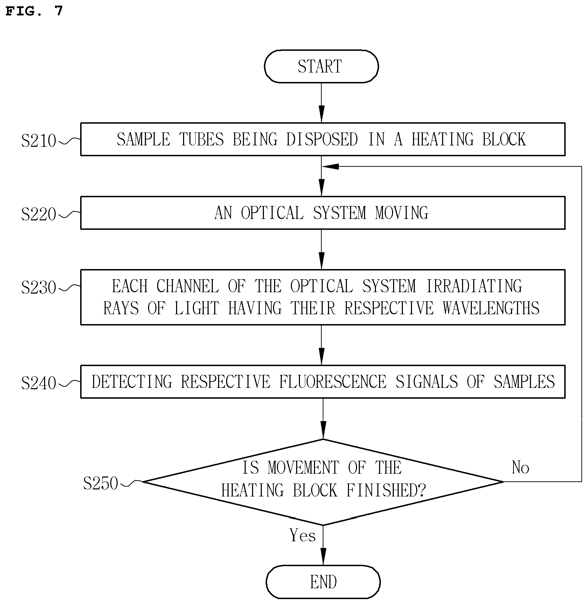

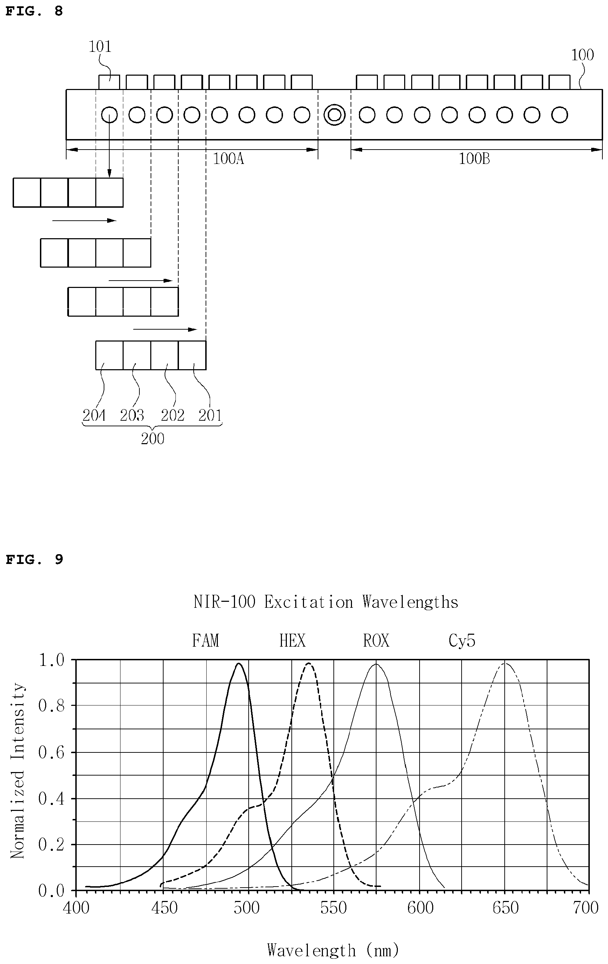

FIG. 7 is a flow chart for describing an operation method of the multi-channel isothermal amplification system according to another exemplary embodiment of the present invention, and FIG. 8 is a view for describing the operation method of the multi-channel isothermal amplification system according to another exemplary embodiment of the present invention.

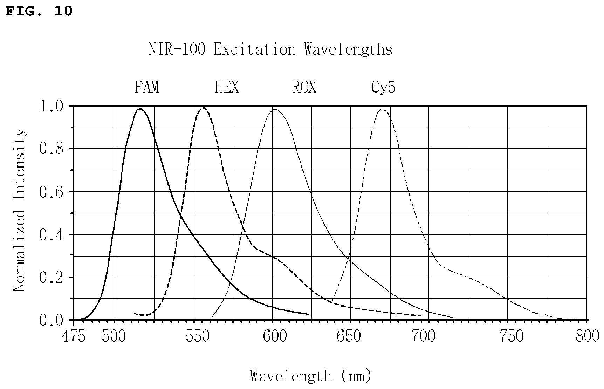

Furthermore, FIG. 9 and FIG. 10 are graphs for describing the operation method of the multi-channel isothermal amplification system according to another exemplary embodiment of the present invention.

Hereinafter, the operation method of the multi-channel isothermal amplification system according to another exemplary embodiment of the present invention is described with reference to FIG. 7 to FIG. 10.

First, the sample tubes 101 are disposed in the holes formed in a line in the heating block 100, respectively S210.

The heating block 100 comprises: the first heating block area 100 in which some of the plurality of holes are formed in a line; and the second heating block area 100B in which the rest of the plurality of holes are formed in a line, and which is disposed to be in a line with the first heating block area 100A.

At this time, the first heating block area 100A and the second heating area 100B may be configured to be spaced apart from each other at an interval as much as one hole area between two holes.

After these sample tubes 101 are disposed, the optical system 200 moves S220, and at this time, each of the channels 201, 202, 203, and 203 of the optical system irradiates rays of light having their respective wavelengths S230, thereby detecting respective fluorescence signals from the samples disposed in the sample tubes 101 S240.

Describing in more detail, the optical system 200 comprising the first channel 201 to the fourth channel 204 moves in the longitudinal direction of the first heating block area 100A and the second heating block area 100B so that the first channel 201 to the fourth channel 204 each cause the rays of light having their respective fixed wavelengths to be successively incident upon the sample tubes 101 inserted into the holes of the first heating block area 100A and the holes of the second heating block area 100B, thereby detecting the respective fluorescence signals of the samples disposed in the sample tubes 101.

That is, the optical system 200 is configured in such a manner that the plurality of channels 201, 202, 203, and 204 are configured as one module so as to move simultaneously, wherein the first channel 201 among the plurality of channels moves in the longitudinal direction of the first heating block area 100A and the second heating block area 100B which are disposed in a line, and causes rays of light having their respective first wavelengths to be successively incident upon the sample tubes inserted into the holes of the first heating block area 100A and the holes of the second heating block area 100B, thereby detecting respective fluorescence signals of the samples disposed in the sample tubes 101, and the second channel 202 among the plurality of channels moves in the longitudinal direction of the first heating block area 100A and the second heating block area 100B which are disposed in a line, and causes rays of light having their respective second wavelengths to be successively incident upon the sample tubes 101 inserted into the holes of the first heating block area 100A and the holes of the second heating block area 100B, thereby detecting respective fluorescent signals of the samples disposed in the sample tubes 101.

Similarly, the third channel 203 among the plurality of channels moves in the longitudinal direction of the first heating block area 100A and the second heating block area 100B which are disposed in a line, and causes rays of light having their respective third wavelengths to be successively incident upon the sample tubes 101 inserted into the holes of the first heating block area 100A and the holes of the second heating block area 100B, thereby detecting respective fluorescence signals of the samples disposed in the sample tubes, and the fourth channel 204 among the plurality of channels moves in the longitudinal direction of the first heating block area 100A and the second heating block area 100B which are disposed in a line, and causes rays of light having their respective fourth wavelengths to be successively incident upon the sample tubes inserted into the holes of the first heating block area 100A and the holes of the second heating block area 100B, thereby detecting respective fluorescent signals of the samples disposed in the sample tubes 101.

Meanwhile, the first heating block area 100A and the second heating block area 100B are spaced apart from each other at an interval as much as the size of one hole area, and when the optical system 200 has detected the respective fluorescence signals of the samples from the first heating block area 100A, the respective fluorescence signals of the samples from the second heating block area 100B are detected in order.

Like this, when the optical system 200 moves, each of the channels 201, 202, 203, and 204 performs a step movement to correspond to each hole, the measurement of samples by each channel 201, 202, 203, or 204 is carried out in a state of the optical system 200 stopping.

Accordingly, as illustrated in FIG. 9, each channel 201, 202, 203, or 204 of the optical system causes the rays of light having their respective wavelengths to be incident upon the sample tubes, thereby detecting the respective fluorescence signals of the samples as shown in FIG. 10.

Like this, the multi-channel isothermal amplification system according to the present invention can obtain accurate result values more rapidly and efficiently through detection of the fluorescence signals using the rays of light having more various wavelengths with regard to the plurality of sample tubes.

As previously described, in the detailed description of the invention, having described the detailed exemplary embodiments of the invention. However, it should be apparent that various modifications can be made without deviating from the scope of the invention. The technical idea of the present invention is not to be construed as limited to the aforesaid exemplary embodiments, but is to be construed within the scope of the claims and their equivalents.

## Claims

1. An operation method of a multi-channel isothermal amplification system, comprising:
a first step in which sample tubes are disposed in holes formed in a line in a heating block, respectively, wherein the heating block comprises a first heating block area in which some of the plurality of holes are formed in a line, and a second heating block area in which the rest of the plurality of holes are formed in a line, which is disposed to be in a line with the first heating block area, wherein the first heating area and the second heating area are spaced apart from each other at an interval as much as one hole area between two holes; and
a second step in which an optical system moves in a longitudinal direction of the first heating block area and the second heating block area which are disposed in a line, and the optical system also causes rays of light having their respective fixed wavelengths to be successively incident upon the sample tubes inserted into the holes of the first heating block area and the holes of the second heating block area, thereby detecting respective fluorescence signals of samples disposed in the sample tubes.

2. The method of claim 1, wherein the optical system comprises four channels consisting of: a luminous source module configured to cause the rays of light to be incident from a lower part of the heating block onto sides of the sample tubes; and a detection module configured to detect the respective fluorescence signals from the samples at a side part of the heating block, and
the second step is performed in such a manner that the optical system consisting of the first channel to the fourth channel moves in the longitudinal direction of the first heating block area and the second heating block area so that the first channel to the fourth cannel each cause the rays of light having their respective fixed wavelengths to be successively incident upon the sample tubes inserted into the holes of the first heating area and the holes of the second heating area, thereby detecting respective fluorescence signals of the samples disposed in the sample tubes.

3. The method of claim 2, wherein the second step is performed in such a manner that the plurality of channels of the optical system move simultaneously,
wherein the first channel among the plurality of channels moves in the longitudinal direction of the first heating block and the second heating block which are disposed in a line, and causes rays of light having their respective first wavelengths to be successively incident upon the sample tubes inserted into the holes of the first heating block area and the holes of the second heating block area, thereby detecting respective fluorescence signals of the samples disposed in the sample tubes,
the second channel among the plurality of channels moves in the longitudinal direction of the first heating block and the second heating block which are disposed in a line, and causes rays of light having their respective second wavelengths to be successively incident upon the sample tubes inserted into the holes of the first heating block area and the holes of the second heating block area, thereby detecting respective fluorescence signals of the samples disposed in the sample tubes,
the third channel among the plurality of channels moves in the longitudinal direction of the first heating block and the second heating block which are disposed in a line, and causes rays of light having their respective third wavelengths to be successively incident upon the sample tubes inserted into the holes of the first heating block area and the holes of the second heating block area, thereby detecting respective fluorescence signals of the samples disposed in the sample tubes, and
the fourth channel among the plurality of channels moves in the longitudinal direction of the first heating block and the second heating block which are disposed in a line, and causes rays of light having their respective fourth wavelengths to be successively incident upon the sample tubes inserted into the holes of the first heating block area and the holes of the second heating block area, thereby detecting respective fluorescence signals of the samples disposed in the sample tubes.

4. A multi-channel isothermal amplification system, comprising:
a heating block in which holes are formed in a line, into the holes are inserted a plurality of sample tubes, respectively; and
an optical system configured to cause rays of light having their respective different wavelengths to be incident upon the sample tubes inserted into the holes of the heating block, thereby detecting respective fluorescence signals from samples disposed in the sample tubes,
wherein the heating block comprises:
a first heating block area in which some of the plurality of holes are formed in a line; and
a second heating block area in which the rest of the plurality of holes are formed in a line, which is spaced apart from the first heating block area at an interval as much as one hole area between two holes in the longitudinal direction of the first heating block area, and which is disposed to be in a line with the first heating block area, and
the optical system moves in the longitudinal direction of the first heating block area and the second heating block area which are disposed in a line, and the optical system also causes the rays of light having their respective fixed wavelengths to be successively incident upon the sample tubes inserted into the holes of the first heating block area and the holes of the second heating block area, thereby detecting the respective fluorescence signals of the samples disposed in the sample tubes.

5. The multi-channel isothermal amplification system of claim 4, further comprising:
a radiant heat plate configured to emit heat from the heating block to the outside; and
a Peltier module disposed at a position between the heating block and the radiant heat plate, and configured to exchange heat between the heating block and the radiant heat plate.

6. The multi-channel isothermal amplification system of claim 4, wherein optical system comprises four channels consisting of: a luminous source module configured to cause the rays of light to be incident from a lower part of the heating block onto sides of the sample tubes; and a detection module configured to detect the fluorescence signals from the samples at a side part of the heating block.

7. The multi-channel isothermal amplification system of claim 6, wherein the radiant heat plate comprises:
a first radiant heat plate disposed at another side part opposite to a side part at which the detection module is disposed, thereby emitting heat from the first heating block area to the outside; and
a second radiant heat plate disposed at the other side part opposite to the side part at which the detection module is disposed, thereby emitting heat from the second heating block area to the outside.

8. The multi-channel isothermal amplification system of claim 6, wherein the luminous source module comprises:
a light-emitting diode (LED) configured to irradiate the rays of light;
an excitation filter configured to filter out the rays of light irradiated from the LED; and
a first lens configured to concentrate the rays of light filtered out by the excitation filter on the samples disposed in the sample tubes.

9. The multi-channel isothermal amplification system of claim 6, wherein the detection module comprises: an emission filter configured to receive the respective fluorescence signals of the samples; a second lens which the fluorescence signals filtered out by the emission filter penetrate; and a light perception sensor configured to receive the fluorescence signals coming in through the second lens.
